# EUROPÄISCHE PATENTSCHRIFT

(11) **EP 2 191 773 B1**
(45) Veröffentlichungstag und Bekanntmachung des Hinweises auf die Patenterteilung: **15.04.2015**
(21) Anmeldenummer: 10156032.4
(22) Anmeldetag: 07.03.2006
(51) Int. Cl.: A61B 5/151, A61B 5/00, A61B 10/00, B01L 3/00, G01N 33/487, G01N 35/00, A61B 5/145

(54) **Bandmagazin für ein Handgerät zur Untersuchung einer Körperflüssigkeit, sowie Handgerät**
Tape magazine for hand-held device for analyzing body fluids, and hand-held device
Magasin-ruban pour un dispositif d'essai portatif pour examiner des liquides organiques, et dispositif d'essai portatif

(30) Priorität: 18.03.2005 DE 102005013685
(43) Veröffentlichungstag der Anmeldung: 02.06.2010
(62) Teilanmeldung aus: 06004546.5
(73) Patentinhaber: F. Hoffmann-La Roche AG, 4070 Basel (CH); Roche Diagnostics GmbH, 68305 Mannheim (DE)
(72) Erfinder: List, Hans, 64754, Hesseneck-Kailbach (DE); Haar, Hans-Peter, Dr., 69168, Wiesloch (DE); Roesicke, Bernd, 68305, Mannheim (DE)
(74) Vertreter: Pfiz, Thomas

(56) Entgegenhaltungen:
- WO-A1-02/100274
- WO-A2-2005/006985
- DE-A1- 19 819 407

## Beschreibung

Die Erfindung betrifft ein Bandmagazin für ein Handgerät zur Untersuchung einer Körperflüssigkeit mit einem Testband, einem Spulenkörper für unverbrauchtes Testband und einem Spulenkörper für verbrauchtes Testband, wobei zumindest ein Spulenkörper zum Vorspulen des Testbandes angetrieben ist und ein handbetätigbarer Transportmechanismus als Bandantrieb vorgesehen ist.

Bei derartigen Bandmagazinen ist es vorgesehen, dass von einem Vorratswickel ein unbenutzter Testbandabschnitt abgezogen und über eine Aufnahmeeinrichtung geführt wird, wo er eine Probe der Körperflüssigkeit aufnimmt. Anschließend wird der nun benutzte Testbandabschnitt auf einem Abfallwickel aufgewickelt. Der Aufnahmeeinrichtung ist eine Detektionseinrichtung zugeordnet, welche die Probe vermisst und das Messergebnis an eine Auswerteeinrichtung weiterleitet.

Bevorzugte werden solche Bandmagazine in Blutzuckermessgeräten für Diabetiker eingesetzt, die auf eine ständige Kontrolle ihrer Blutzuckerwerte angewiesen sind. Das Testband ermöglicht nach dem Applizieren von Kapillarblut, beispielsweise aus der Fingerbeere, einen geräteinternen Blutzuckernachweis. Hierfür ist eine Vielzahl von Testabschnitten bzw. Testfeldern auf dem Testband fortlaufend angeordnet. Ein unverbrauchter Testabschnitt wird durch einen Bandvorlauf in eine aktive Position gebracht. Dann wird das Kapillarblut appliziert und analysiert. Für eine einfache Dosierung geringster Blutmengen und für eine möglichst genaue Positionierung bezüglich der Detektionseinrichtung wird das Testband über eine gehäuseinterne Umlenkspitze gelenkt. Dabei besteht die Gefahr von Fehlmessungen, wenn das Testband von der Umlenkspitze herunterrutscht. Für eine erfolgreiche Messung muss das Testband an einer wohldefinierten Stelle verbleiben und glatt anliegen. Dabei sollte ein vorbestimmter Abstand zur Detektionseinrichtung eingehalten werden. Dies gilt mindestens so lange, bis die Messung abgeschlossen ist. Eine weitere Herausforderung liegt darin, dass das Testband sehr empfindlich gegen Kontamination ist. Der unbenutzte Bereich des Testbandes sollte daher gegenüber dem benutzten Bereich räumlich getrennt und auch von äußeren Einflüssen abgeschirmt werden, welche die Funktion des Testbandes beeinträchtigen könnten. Eine direkte Antriebskopplung zwischen der Vorratseinheit und der Abfalleinheit ist daher nur schwer möglich.

Ferner sind die vorbekannten Handgeräte für einen Dauergebrauch vorgesehen, während das Bandmagazin ausgewechselt wird. Die Handgeräte sind daher relativ groß und, nicht zuletzt wegen der aufwendigen Gerätetechnik, recht fertigungsintensiv.

Aus der DE 198 19 407 A1 ist ein Teststreifenbehälter für Meßgeräte insbesondere zur Bestimmung von Blutzuckerwerten bekannt, wobei in diesem Zusammenhang erwähnt wird, dass die Teststreifen so aneinandergereiht sind, dass sie ein Band bilden. Welches ähnlich dem Band in iner Tonbandkassette gespult werden kann, und dass das Teststreifenband durch eine Mechanik automatisch oder manuell gespult wird.

Die WO 02/100274 A1 schlägt für ein integriertes Körperflüssigkeits-Sammel- und Testbandgerät eine mechanische Vorschubeinrichtung vor, um das Testmedium zu befördern, wobei ein komplizierter geräteseitiger Getriebemechanismus vorgesehen ist, der eine Stechbewegung eines Stechteils zugleich in eine Bandbewegung umsetzen soll, wobei dann noch ein Freilauf bei der Stech-Rückbewegung erforderlich ist.

Die Aufgabe besteht darin, ein Handgerät bzw. ein Bandmagazin dafür in kompakter und herstellungstechnisch günstiger Bauform bereit zu stellen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Dementsprechend wird vorgeschlagen, dass der Transportmechanismus einen mit mindestens einem Spulenkörper gekoppelten Betätigungshebel für die Handbetätigung aufweist.

Bevorzugt ist ein Betätigungshebel vorgesehen, der über ein Vorschuborgan, insbesondere eine Klinke in ein Vorschubelement (z.B. Capstan oder Indexrad) eingreift, derart, dass das Testband mechanisch bewegbar ist. Damit kann auf eine Stromversorgung über Batterien verzichtet werden. Die für den eigentlichen Messvorgang notwendige Energie kann durch die manuelle Betätigung insbesondere mittels induktivem Generator oder piezoelektrisch erzeugt und beispielsweise in einem Kondensator oder Hochleistungskondensator (Supercap) zwischengespeichert werden. Bevorzugt bildet das Bandmagazin zugleich ein als Einmalartikel vorgesehenes Handgerät, ein so genanntes Disposable, welches aufgrund der kostengünstigen Bauweise nach Verbrauch des Testbandes verworfen werden kann.

Der Vorschubmechanismus kann beispielsweise durch eine Ratsche gebildet sein, welche in an der Vorratseinheit und/oder Abfalleinheit ausgebildete Stufen eingreift. Eine solche Konstruktion ist robust und einfach zu bedienen.

Der Vorschub des Testbandes ist vorteilhafterweise durch eine Perforation derart synchronisiert, dass bei mindestens einmaliger Betätigung des Hebels ein Testelement für eine Messung bereit steht, so dass die Messung besonders zuverlässig und mit großer Genauigkeit erfolgt. Zur weiteren Verbesserung der Vorschubgenauigkeit können auf dem Band Referenzfelder vorgesehen sein.

Insbesondere sind alle offenbarten Ausführungsformen von Bandmagazinen und Handgeräten miteinander kombinierbar. Dies gilt für allem für ein Handgerät mit elektronischen Bauteilen auf der Basis von Polymerelektronik kombiniert mit einem mechanischen Antrieb für das Testband.

Im Folgenden wird die Erfindung anhand der in der Zeichnung schematisch dargestellten Ausführungsbeispiele näher erläutert. Es zeigen:
- Fig. 1: ein erstes Ausführungsbeispiel eines erfindungsgemäßen Bandmagazins im Schnitt;
- Fig. 2: ein zweites Ausführungsbeispiel eines erfindungsgemäßen Bandmagazins in einer teilweise geschnittenen Seitenansicht;
- Figur 3: ein drittes Ausführungsbeispiel eines erfindungsgemäßen Bandmagazins in einer teilweise geschnittenen Seitenansicht;
- Figur 4: ein Ausführungsbeispiel eines mechanisch betriebenen Bandgeräts in einer perspektivischen Darstellung mit teilweise geöffnetem Gehäuse;
- Figur 5: das Gerät gemäß Figur 4 mit geschlossenem Gehäuse.

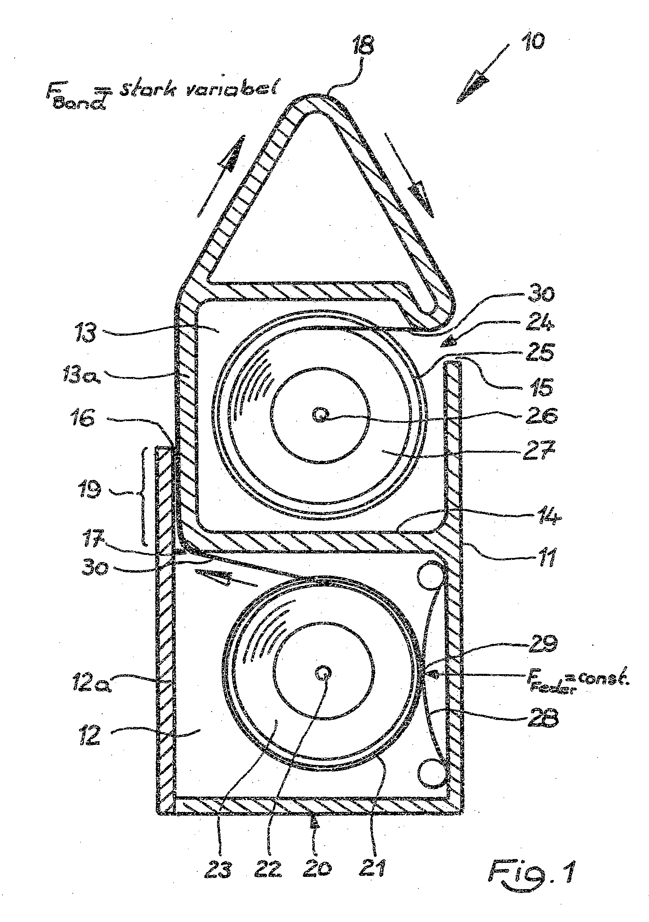

Die Figuren 1 bis 3 zeigen verschiedene Ausführungsbeispiele eines erfindungsgemäßen Bandmagazins, bei dem das Testband unter Zugspannung gehalten ist. Figur 1 zeigt ein Bandmagazin 10 mit einem Gehäuse 11. Das Gehäuse 11 ist unterteilt in eine erste Aufnahme 12 für eine Vorratseinheit 20 und eine zweite Aufnahme 13 für eine Abfalleinheit 24. Die Aufnahmen 12, 13 sind durch eine Trennwand 14 voneinander getrennt, so dass die Vorratseinheit 20 von der Abfalleinheit 24 separiert ist. Je eine Seitenwand 12a, 13a 12, 13 überlappen sich im 19 bildet 13a der Aufnahmen 12, 13 überlappen sich im Bereich 19 und bilden eine Öffnung 16, die mit einer Dichtung 17 versehen ist. Eine eitere Öffnung 15 ist in der Aufnahme 13 für die Abfalleinheit 24 vorgesehen. In das Gehäuse 10 ist eine Umlenkspitze 18 für das Testband 30 integriert.

Die Vorratseinheit 20 weist ein Spulengehäuse 21 auf, welches ein um eine Spule 22 zu einem Vorratswickel 23 aufgewickeltes unverbrauchtes Testband 30 aufnimmt. In vergleichbarer Weise ist die Abfalleinheit 24 mit einem Spulengehäuse 25 ausgestattet, welches das um eine Spule 26 zu einem Abfallwickel 27 aufgewickelte verbrauchte Testband aufnimmt. Die Spule 26 wird von einem Antrieb (nicht dargestellt) angetrieben. Das Testband 30 ist in hintereinander liegende Testbereiche aufgeteilt. Wird der Antrieb betätigt, so wird das frische Testband 30 vom Vorratswickel 23 abgespult und aus der Aufnahme 12 durch die Öffnung 16 hindurch zu der Umlenkspitze 18 geführt, wo ein einzelner Testbereich frei nach außen zu liegen kommt und eine Testflüssigkeit, beispielsweise einen Blutstropfen, aufnehmen kann. Die Testflüssigkeit wird von einer Detektionseinheit (nicht dargestellt) vermessen. Bei nochmaligem Betätigen des Antriebs wird das Testban 30 weiter transportiert. Das verbrauchte Testband 30 tritt durch die Öffnung 15 in die Aufnahme 13 ein und wird auf dem Abfallwickel 27 aufgewickelt.

An einer Innenwand der Aufnahme 12 für die Vorratseinheit 20 ist eine Blattfeder 28 vorgesehen, die am Kontaktpunkt 29 das Spulengehäuse 21 beaufschlagt. Die Blattfeder 28 ist mit einer konstanten Federkraft F_{Feder} vorgespannt. Daher muss das Testband 30 gegen das entsprechende Bremsmoment mit einer gewissen Bandzugkraft F_{Band} abgespult werden. Diese Bandzugkraft nimmt mit abnehmendem Radius des Vorratswickels 23 zu.

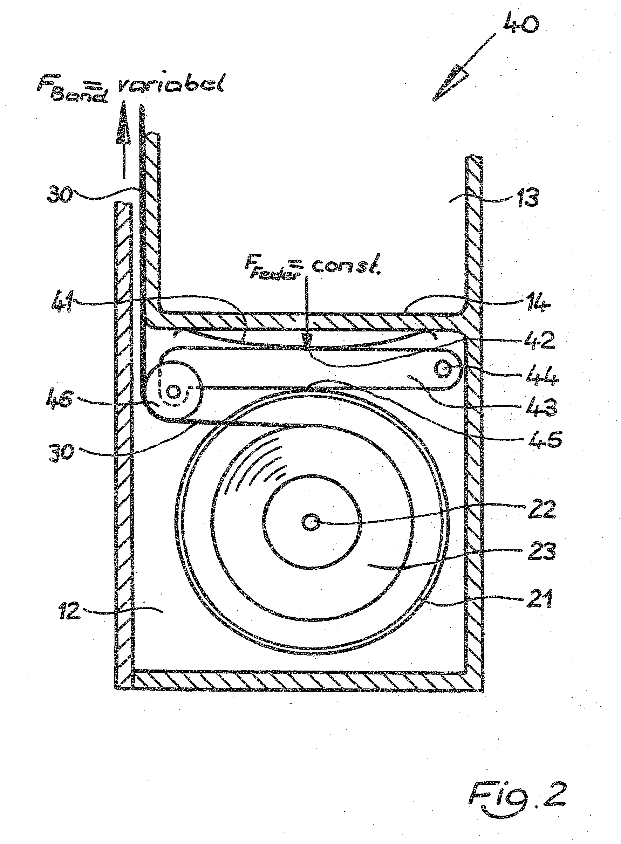

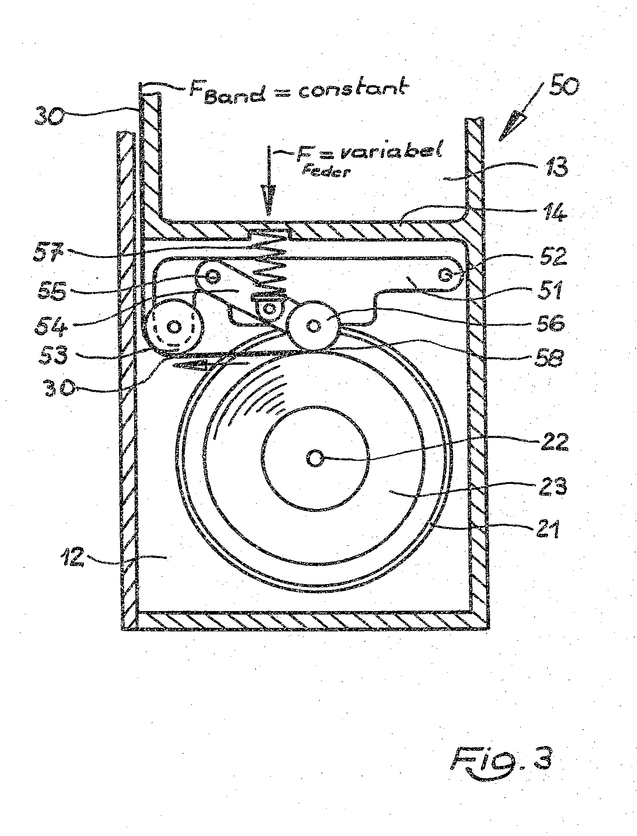

Die Figuren 2 und 3 zeigen zwei weitere Ausführungsbeispiele eines erfindungsgemäßen Bandmagazins 40, 50, welche sich von dem soeben beschriebenen Bandmagazin 10 nur in der Ausgestaltung der Bremsmittel unterscheiden. Gleiche Bauteile sind daher mit gleichen Bezugszeichen versehen.

Das in Figur 2 gezeigte Bandmagazin 40 weist an einer Innenwand der Aufnahme 12 unterhalb der Trennwand 14 ebenfalls eine Blattfeder 41 auf, die mit einer konstanten Federkraft F_{Feder} vorgespannt ist. An der Innenwand der Aufnahme 12 ist ferner ein Schwinghebel 43 um eine Achse 44 drehbar angelenkt. Der Schwinghebel 43 ist an seinem freien Ende mit einer drehbaren Umlenkrolle 46 versehen, über welche das Testband 30 geführt ist. Der Schwinghebel 43 ist zwischen der Blattfeder 41 und dem Spulengehäuse 21 angeordnet und verläuft tangential zum Spulengehäuse 21. Der Schwinghebel 43 berührt die Blattfeder 41 in einem Kontaktpunkt 42 und das Spulengehäuse 21 in einem Kontaktpunkt 45. Der Schwinghebel 43 wird von der Blattfeder 41 mit der konstanten Federkraft F_{Feder} beaufschlagt. Demzufolge wird auch das Spulengehäuse 21 vom Schwinghebel 43 mit einer entsprechenden Kraft beaufschlagt.

Wenn der Antrieb für das Testband 30 betätigt wird, dann muss es gegen das auf das Spulengehäuse 21 wirkende Bremsmoment mit einer gewissen Bandzugkraft F_{Band} abgespult werden. Die Bandzugskraft greift am langen Hebelarm des Schwinghebels 43 über die Umlenkrolle 46 an und entlastet den Kontaktpunkt 45 abhängig von der Bandzugkraft. Mit abnehmendem Durchmesser des Vorratswickels 23 muss also eine geringere Bandzugkraft zum Abspulen des Testbands aufgebracht werden als bei dem in Figur 1 dargestellten Ausführungsbeispiel.

Das in Figur 3 dargestellte Ausführungsbeispiel eines Bandmagazins 50 weist an der Innenwand der Aufnahme 12 unterhalb der Trennwand 14 ebenfalls einen Schwinghebel 51 auf, der um eine Achse 52 drehbar an der Innenwand angelenkt ist. Der Schwinghebel 51 ist an seinem freien Ende auch mit einer drehbaren Umlenkrolle 53 versehen, über welche das Testband 30 geführt ist. Der Schwinghebel 51 verläuft ebenfalls tangential zum Spulengehäuse 21 und berührt das Spulengehäuse 21 in einem hier nicht sichtbaren Kontaktpunkt.

Am Schwinghebel 51 ist eine um eine Achse 55 drehbare Schwinge 54 angelenkt. Die Schwinge 54 ist an ihrem freien Ende mit einer Abtastrolle 56 versehen, welche auf dem Umfang des Vorratswickels 23 an einem Kontaktpunkt 58 aufliegt. Eine mit einer bestimmten Federkraft F_{Feder} vorgespannte Druckfeder 57 stützt sich an der Trennwand 14 und an der Schwinge 54 ab, so dass die Schwinge 54 mit dieser Federkraft beaufschlagt ist.

Wenn der Antrieb für das Testband 30 betätigt wird, dann muss es gegen die auf das Spulengehäuse 21 wirkende Kraft mit einer gewissen Bandzugkraft F_{Band} abgespult werden. Diese Bandzugskraft greift am langen Hebelarm des Schwinghebels 51 an und entlastet den Kontaktpunkt zwischen dem Schwinghebel 51 und dem Spulengehäuse 21 abhängig von der Bandzugkraft. Gleichzeitig läuft die Abtastrolle 56 der Schwinge 54 auf dem Umfang des Vorratswickels 23. Mit abnehmendem Radius des Vorratswickel 23 wandert der Abtastrolle 56 in Richtung der Spule 22, so dass die Druckfeder 57 sich mit abnehmendem Radius des Vorratswickels 23 entspannt. Damit wird der Kontaktpunkt zwischen dem Schwinghebel 51 und dem Spulengehäuse 21 in Abhängigkeit vom Radius des Vorratswickels 23 entlastet. Als Resultat bleibt die vom Antrieb aufzubringende Bandzugskraft F_{Band} mit abnehmendem Radius des Vorratswickels 23 konstant.

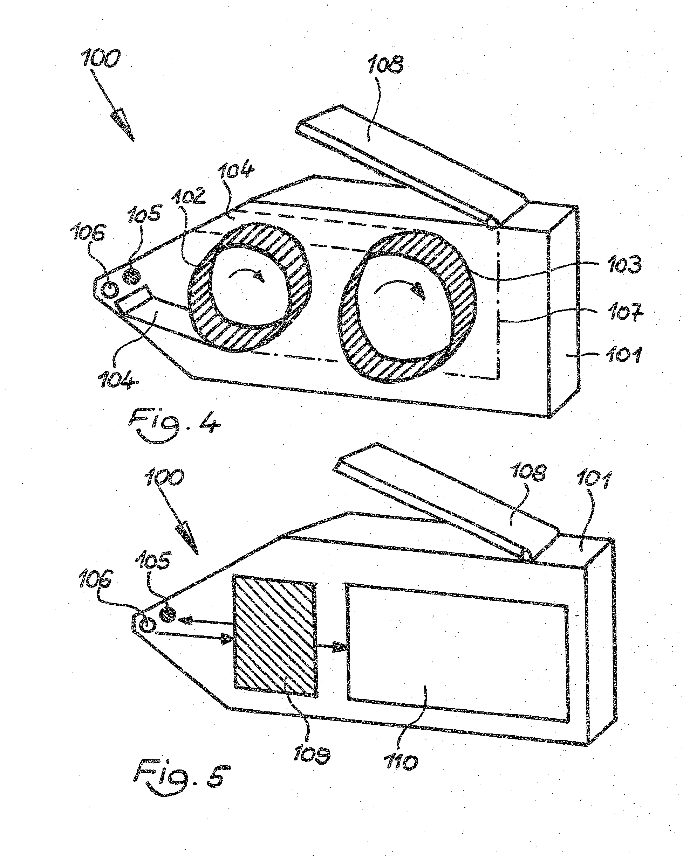

Die Figuren 4 und 5 zeigen eine Kombination aus einem Handgerät mit elektronischen Bauteilen auf Polymerbasis und einem mechanischen Antrieb für das Testband.

Das Handgerät 100 ist ein Einweggerät, ein sogenanntes Disposable. Es weist ein Gehäuse 101 aus Kunststoff auf, in welche zwei Spulenkörper 102, 103 angeordnet sind. Auf den Spulenkörpern 102, 103 ist ein Testband 104 mit aufeinander folgenden Testfeldern aufgewickelt. Im Gehäuse 101 ist ferner in photooptischer Sensor 105 in räumlicher Nähe zu einem Messort 106 vorgesehen. Am Messort 106 ist das Testban 104 von außen zugänglich, um eine Probenflüssigkeit, beispielsweise Blut zur Blutzuckerbestimmung, aufzunehmen. Der Bereich zwischen Sender und Empfänger des Sensors und dem Testband kann durch optischen Strahlengang oder Lichtleiter überbrückt werden. Der vom Sensor 105 aufgenommene Messwert wird in eine Auswerteeinheit 109 übertragen. Dort wird ein Anzeigewert, beispielsweise der Blutzuckergehalt, berechnet.

Der photooptische Sensor besteht aus mindestens einer LED geeigneter Wellenlänge, vorzugsweise OLED, kombiniert mit einer oder mehreren organischen Photodioden (Multiphotometerprinzip). Auch LEDs mit Mehrfachwellenlängen sind denkbar.

Die Auswerteeinheit 109 enthält Verstärker, AD-Konverter, Rechenwerk, Schaltwerk, Datenspeicher, Energieversorgung und Schnittstellen und ist mit einer Anzeigeeinheit 110 verbunden, die den ermittelten Anzeigewert auf einem Display darstellt. Die Anzeigeeinheit kann in an sich bekannter Weise so ausgebildet sein, dass eine Anzeige bis zum nächsten Messvorgang auch ohne Energieversorgung aufrechterhalten werden kann, beispielsweise durch Verwendung sog. "elektronischer Tinten".

Der Datenspeicher in der Auswerteeinheit kann als ROM oder EEPROM ausgebildet sein. Er wird hauptsächlich zur Speicherung chargenspezifischer Daten benötigt, die bei der Produktion des Disposables ermittelt und darauf hinterlegt werden. Die Datenübermittlung geschieht mittels Kontaktschnittstellen oder RF-ID-Transponder. Mit einem EEPROM wäre auch ein elektronisches Testfeld-Zählwerk realisierbar.

Die elektronischen Bauteile dieses Handgeräts sind an sich bekannte polymerelektronische Bauteile. Solche Bauteile sind in der WO 2004/044571 A1 beschrieben, deren diesbezüglicher Inhalt hiermit in die Offenbarung der vorliegenden Patentanmeldung aufgenommen wird. Die Verwendung derartiger Bauteile ermöglicht es, sämtliche notwendigen elektronischen Komponenten in ein Magazingehäuse zu integrieren, so dass das resultierende Bandmagazin zugleich ein voll funktionsfähiges und sehr handliches Einweg-Handgerät darstellt. Ein derartiges Einweg-Handgerät ist klein und leicht, kostengünstig und einfach zu bedienen. Das Wechseln des Bandmagazins entfällt. Der Weg für eine weitergehende Miniaturisierung von tragbaren Handgeräten ist offen. Die aufwendige Konstruktion von Schnittstellen zwischen Bandmagazin und Handgerät entfällt ebenfalls.

Alle elektronischen Bauteile auf Polymerbasis können in an sich bekannter Weise auf geeignete Ausformungen des Gehäuse 101 des Handgeräts 100 aufgedruckt werden.

Die Energieversorgung erfolgt mit Hochleistungskondensatoren (Supercap), beispielsweise kombiniert mit Solarzelle. Aufgrund der geringen erreichbaren Energiedichte empfiehlt es sich, die Spulenkörper 102, 103 des beschriebenen Einweg-Handgeräts manuell anzutreiben.

Die Spulenkörper 102, 103 sind hierfür mit Zähnen oder Stufen versehen. In diese Zähne oder Stufen greift eine hier nur angedeutete Vorschubklinke 107 ein. Die Vorschubklinke 107 ist mit einem außen am Gehäuse vorgesehenen Hebel 108 verbunden. Der Vorschub des Testbandes 104 erfolgt durch Betätigen des Hebels. Dabei werden die Spulenkörper 102, 103 genau so weit bewegt, dass ein frisches Testfeld des Testbandes 104 am Messort 106 von außen zugänglich ist. Zur Synchronisation der Bewegungen von Spulenkörpern 102, 103 und Testband 104 ist letzteres perforiert, so dass auf den Spulenkörpern 102, 103 angeordnete Zähne (nicht dargestellt) in die Perforation eingreifen. Die Testfelder können auch so auf dem Testband beabstandet verteilt sein, dass bei einer ersten Betätigung des Hebels 108 ein frisches Testfeld am Messort 106 zugänglich gemacht wird. Mit einer zweiten Betätigung des Hebels 108 wird das nun verbrauchte Testfeld vom Messort 106 weg bewegt, ohne dass dabei sofort ein neues Testfeld erscheint. Dies geschieht erst bei einer neuerlichen Betätigung des Hebels 108.

Mit der Betätigung des Hebels 108 kann auch die für die Messung notwendige Energie von wenigen Milliwatt in an sich bekannter Weise generiert und beispielsweise in einem Kondensator bzw. Supercap zwischengespeichert werden. Zur generatorischen oder piezoelektrischen Energiegewinnung kann ein mechanischer Zwischenspeicher in Form einer Feder vorgesehen sein, der es erlaubt, die unterschiedlichen Zeitkonstanten anzupassen.

Durch Verzicht auf einen elektrochemischen Energiespeicher wird ein besonders umweltfreundliches Gerätekonzept realisierbar.

## Patentansprüche

1. Bandmagazin (100) zur Untersuchung einer Körperflüssigkeit mit einem Testband (104), einem Spulenkörper (102) für unverbrauchtes Testband und einem Spulenkörper (103) für verbrauchtes Testband, wobei zumindest ein Spulenkörper (103) zum Vorspulen des Testbandes angetrieben ist und ein handbetätigbarer Transportmechanismus (107,108) als Bandantrieb vorgesehen ist, **dadurch gekennzeichnet, dass** der Transportmechanismus (107,108) einen mit mindestens einem Spulenkörper (102,103) gekoppelten Betätigungshebel (108) für die Handbetätigung aufweist.

2. Bandmagazin nach Anspruch 1, **dadurch gekennzeichnet, dass** der Betätigungshebel (108) außen an einem die Spulenkörper (102,103) enthaltenden Gehäuse (101) angeordnet ist.

3. Bandmagazin nach Anspruch 1 oder 2, **dadurch gekennzeichnet, dass** der Betätigungshebel (108) über ein Vorschuborgan, insbesondere eine Klinke (107) in ein Vorschubelement, insbesondere ein Capstan oder Indexrad eingreift, so dass das Testband (104) mechanisch bewegbar ist.

4. Bandmagazin nach einem der Ansprüche 1 bis 3, **dadurch gekennzeichnet, dass** der Transportmechanismus (107,108) eine Rätsche aufweist, welche in an mindestens einem Spulenkörper (102,103) ausgebildete Stufen oder Zähne eingreift.

5. Bandmagazin nach einem der Ansprüche 1 bis 4, **dadurch gekennzeichnet, dass** das Testband (104) mit aufeinander folgenden Testfeldem versehen ist und dass die Testfelder durch Betätigung des Transportmechanismus (107,108) an einem Messort (106) positionierbar sind.

6. Bandmagazin nach einem der Ansprüche 1 bis 5, **dadurch gekennzeichnet, dass** der Vorschub des Testbandes (104) vorzugsweise durch eine Perforation derart synchronisiert ist, dass bei mindestens einmaliger Betätigung Transportmechanismus (107,108) ein Testelement für eine Messung bereit steht.

7. Bandmagazin nach einem der Ansprüche 1 bis 6, **dadurch gekennzeichnet, dass** der Transportmechanismus (107,108) einen Energiewandler zur Umsetzung mechanischer in elektrische Energie für die Energieversorgung einer Geräteelektronik aufweist.

8. Bandmagazin nach einem der Ansprüche 1 bis 7, **dadurch gekennzeichnet, dass** elektrische Energie für einen Messvorgang durch manuelle Betätigung, insbesondere induktiv oder piezoelektrisch, und/oder durch Solarzellen erzeugbar ist und gegebenenfalls in einem Kondensator speicherbar ist.

9. Bandmagazin nach einem der Ansprüche 1 bis 8, **dadurch gekennzeichnet, dass** es zugleich ein als Einmalartikel vorgesehenes Handgerät bildet.

10. Handgerät zur Untersuchung einer Körperflüssigkeit umfassend ein Bandmagazin nach einem der vorhergehenden Ansprüche.

## Claims

1. Tape magazine (100) for analysing a body fluid comprising a test tape (104), a reel body (102) for unused test tape and a reel body (103) for used test tape, wherein at least one reel body (103) is driven in order to forward the test tape and a hand-operated transport mechanism (107, 108) is provided as a tape drive, **characterized in that** the transport mechanism (107, 108) has an operating lever (108) for the hand operation that is coupled to at least one reel body (102, 103).

2. Tape magazine according to claim 1, **characterized in that** the operating lever (108) is arranged at the outside of a housing (101) containing the reel bodies (102, 103).

3. Tape magazine according to claim 1 or 2, **characterized in that** the operating lever (108) engages via a feed member, in particular a pawl (107), into an advancing element, in particular a capstan or an index wheel, in such a manner that the test tape (104) can be moved mechanically.

4. Tape magazine according to claim 1 to 3, **characterized in that** the transport mechanism (107, 108) has a ratchet that engages in steps or teeth formed in at least one reel body (102, 103).

5. Tape magazine according to one of claims 1 to 4, **characterized in that** the test tape (104) is provided with consecutive test fields, and that the test fields can be positioned at a measuring site (106) by operation of the transport mechanism (107, 108).

6. Tape magazine according to one of claims 1 to 5, **characterized in that** the feed of the test tape (104) is synchronized preferably by a perforation in such a manner that when the transport mechanism (107, 108) is actuated at least once, a test element is ready for a measurement.

7. Tape magazine according to one of claims 1 to 6, **characterized in that** the transport mechanism (107, 108) has an energy transducer to convert mechanical into electrical energy in order to supply energy to a device electronics.

8. Tape magazine according to one of claims 1 to 7, **characterized in that** electrical energy required for a measuring process can be generated by manual actuation, particularly inductive or piezo-electrically, and/or by solar cells, and if necessary can be stored in a capacitor.

9. Tape magazine according to one of claims 1 to 8, **characterized in that** it also forms a hand-held device designed as a single-use article.

10. Hand-held device for analyzing a body fluid comprising a tape magazine of any one of the previous claims.

## Revendications

1. Magasin-bande (100) pour l'examen d'un fluide corporel comprenant une bande test (104), un corps de bobine (102) pour la bande test non utilisée et un corps de bobine (103) pour la bande test utilisée, au moins un corps de bobine (103) étant entraîné pour avancer la bande test et un mécanisme de transport (107, 108), actionnable manuellement, étant prévu comme entraînement de bande, **caractérisé en ce que** le mécanisme de transport (107, 108) comporte un levier d'actionnement (108), couplé à au moins un corps de bobine (102, 103), pour l'actionnement manuel.

2. Magasin-bande selon la revendication 1, **caractérisé en ce que** le levier d'actionnement (108) est disposé à l'extérieur d'un boîtier (101) contenant les corps de bobines (102, 103).

3. Magasin-bande selon la revendication 1 ou 2, **caractérisé en ce que** le levier d'actionnement (108) s'engage au moyen d'un organe d'avance, en particulier d'un cliquet (107), dans un élément d'avance, en particulier un cabestan ou une roue indexée, de sorte que la bande test (104) est déplaçable mécaniquement.

4. Magasin-bande selon l'une des revendications 1 à 3, **caractérisé en ce que** le mécanisme de transport (107, 108) comporte un rochet, lequel s'engage dans des crans ou des dents réalisés sur au moins un corps de bobine (102, 103).

5. Magasin-bande selon l'une des revendications 1 à 4, **caractérisé en ce que** la bande test (104) est dotée de zones test successives et **en ce que** les zones test peuvent être positionnées sur un lieu de mesure (106) par actionnement du mécanisme de transport (107, 108).

6. Magasin-bande selon l'une des revendications 1 à 5, **caractérisé en ce que** l'avance de la bande test (104) est synchronisée de préférence par une perforation, de sorte que lorsque le mécanisme de transport (107, 108) est actionné au moins une fois, un élément test est prêt pour une mesure.

7. Magasin-bande selon l'une des revendications 1 à 6, **caractérisé en ce que** le mécanisme de transport (107, 108) comporte un convertisseur d'énergie permettant de convertir l'énergie mécanique en énergie électrique pour l'alimentation de l'électronique de l'appareil.

8. Magasin-bande selon l'une des revendications 1 à 7, **caractérisé en ce que** l'énergie électrique pour une opération de mesure peut être produite par actionnement manuel, notamment inductif ou piézoélectrique, et/ou par piles solaires et peut le cas échéant être stockée dans un condensateur.

9. Magasin-bande selon l'une des revendications 1 à 8, **caractérisé en ce qu'**il constitue en même temps un appareil portatif prévu en tant qu'article à usage unique.

10. Appareil portatif pour l'examen d'un fluide corporel comprenant un magasin-bande selon l'une des revendications précédentes.
